(19) **Europäisches Patentamt / European Patent Office / Office européen des brevets**

(11) **EP 4 327 799 A1**

(12) # EUROPEAN PATENT APPLICATION

(43) Date of publication:
**28.02.2024 Bulletin 2024/09**

(21) Application number: **23192091.9**

(22) Date of filing: **18.08.2023**

(51) International Patent Classification (IPC):
**A61K 8/37** (2006.01)    **A61Q 17/04** (2006.01)

(52) Cooperative Patent Classification (CPC):
**A61K 8/375; A61Q 17/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.08.2022 TW 111131376**

(71) Applicant: **Patech Fine Chemicals Co., Ltd.**
**Taipei City 106 (TW)**

(72) Inventors:
• **SHIH, Hou-Kuang**
  **507 Xianxi Township (TW)**

• **LIANG, An-Hung**
  **507 Xianxi Township (TW)**
• **PAN, Yu-Zih**
  **507 Xianxi Township (TW)**
• **LIN, Chia-Ying**
  **507 Xianxi Township (TW)**
• **HUNG, Jung-Tsung**
  **507 Xianxi Township (TW)**
• **TSAIH, Jeng-Shiang**
  **507 Xianxi Township (TW)**

(74) Representative: **V.O.**
**P.O. Box 87930**
**2508 DH Den Haag (NL)**

(54) **EMULSION COMPONENT AND OIL-IN-WATER SUNSCREEN PRODUCT AND OIL-IN-WATER SUNSCREEN LOTION INCLUDING THE SAME**

(57)    An emulsion component includes a polyglycerol ester-base emulsifier and a polyglycerol ester-based co-emulsifier. The polyglycerol ester-base emulsifier has a hydrophilic-lipophilic balance value greater than 10, and is formed by reacting a first polyglycerol having a degree of polymerization ranging from 4 to 20 with a first acid. The polyglycerol ester-based coemulsifier has a hydrophilic-lipophilic balance value not greater than 10, and is formed by reacting a polyol with a second acid. The polyol is selected from the group consisting of a glycerol and a second polyglycerol having a degree of polymerization ranging from 2 to 10. In addition, a weight ratio of the polyglycerol ester-based emulsifier and the polyglycerol ester-based coemulsifier ranges from 0.75 to 8. An oil-in-water sunscreen product including the emulsion component and an oil-in-water sunscreen lotion including the emulsion component are also provided.

EP 4 327 799 A1

**Description**

The disclosure relates to an emulsion component, and an oil-in-water sunscreen product and an oil-in-water sunscreen lotion including the same.

CN 103585040B discloses an emulsion composition, which includes a coemulsifier consisting of behenyl alcohol, polyglyceryl-10 pentastearate and sodium stearoyl lactylate, and an alkyl polyvinylpyrrolidone. The coemulsifier is present in an amount ranging from 1.5 wt% to 4 wt%, and the alkyl polyvinylpyrrolidone is present in an amount ranging from 1 wt% to 6 wt%, based on 100 wt% of the emulsion composition. The emulsion composition may further contain an oil/fat material present in an amount ranging from 3 wt% to 40 wt%, a thickening agent present in an amount ranging from 0.1 wt% to 4 wt%, a preservative, and water. The emulsion composition of CN 103585040B may be used for preparing a waterproof liquid crystal cosmetic (such as a foundation cream, a sunscreen lotion, and a skin care lotion), and by having the coemulsifier and the alkyl polyvinylpyrrolidone, the emulsion composition of CN 103585040B may render the waterproof liquid crystal cosmetic to have a good water-resistance effect.

However, even though the waterproof liquid crystal cosmetic prepared from the emulsion composition of CN 103585040B has such good water-resistance effect, its sun protection factor is not high enough to adequately provide sunlight protection. Furthermore, the alkyl polyvinylpyrrolidone is a chemical compound, which does not satisfy a current market demand for natural ingredients and is not conducive to sustainable development of the cosmetic market.

In view of the above, there is still a need for those skilled in the art to develop an emulsion component that may be used for preparing a cosmetic having both water-resistance effect and a high sun protection factor while possessing completely natural ingredients.

According to an aspect of the disclosure, there is provided an emulsion component that can alleviate at least one of the drawbacks of the prior art according to claim 1.

According to an aspect of the disclosure, there is provided an oil-in-water sunscreen product that can alleviate at least one of the drawbacks of the prior art according to claim 6.

According to an aspect of the disclosure, there is provided an oil-in-water sunscreen lotion that can alleviate at least one of the drawbacks of the prior art according to claim 8.

It is to be understood that, if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to," and that the word "comprises" has a corresponding meaning.

Unless otherwise defined, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

<Emulsion component>

The present disclosure provides an emulsion component that includes:

a polyglycerol ester-based emulsifier having a hydrophilic-lipophilic balance value greater than 10, the polyglycerol ester-based emulsifier being formed by reacting a first polyglycerol having a degree of polymerization ranging from 4 to 20 with a first acid, and

a polyglycerol ester-based coemulsifier having a hydrophilic-lipophilic balance value not greater than 10, the polyglycerol ester-based coemulsifier being formed by reacting a polyol with a second acid, the polyol being selected from the group consisting of a glycerol and a second polyglycerol having a degree of polymerization ranging from 2 to 10;

wherein a weight ratio of the polyglycerol ester-base emulsifier and the polyglycerol ester-based coemulsifier in decimal form ranges from 0.75 to 8.

In certain embodiments, the weight ratio of the polyglycerol ester-based emulsifier and the polyglycerol ester-based coemulsifier in decimal form may range from 0.75 to 7. In certain embodiments, the weight ratio of the polyglycerol ester-based emulsifier and the polyglycerol ester-based coemulsifier in decimal form may range from 0.75 to 6. In certain embodiments, the weight ratio of the polyglycerol ester-based emulsifier and the polyglycerol ester-based coemulsifier in decimal form may range from 0.75 to 5. In certain embodiments, the weight ratio of the polyglycerol ester-based emulsifier and the polyglycerol ester-based coemulsifier in decimal form may range from 0.75 to 4. In certain embodiments, the weight ratio of the polyglycerol ester-based emulsifier and the polyglycerol ester-based coemulsifier in decimal form may range from 0.75 to 3. In certain embodiments, the weight ratio of the polyglycerol ester-based emulsifier and the polyglycerol ester-based coemulsifier in decimal form may range from 0.75 to 2. In certain embodiments, the weight ratio of the polyglycerol ester-based emulsifier and the polyglycerol ester-based coemulsifier in decimal form may range from 1 to 2, such that the emulsion component imparts a better degree of water resistance to an oil-in-water sunscreen cosmetic.

In certain embodiments, the polyglycerol ester-based emulsifier may have a hydrophilic-lipophilic balance value greater than 10 and not greater than 20. In certain embodiments, the polyglycerol ester-based emulsifier may have a hydrophilic-lipophilic balance value greater than 10 and smaller than 18. In certain embodiments, the polyglycerol ester-based emulsifier may have a hydrophilic-lipophilic balance value greater than 11 and smaller than 17.

In certain embodiments, the first polyglycerol may have a degree of polymerization ranging from 4 to 10. In certain embodiments, the first acid may be a fatty acid or an aromatic acid. In addition, the fatty acid may or may not include a substituent. Examples of the substituent may include, but are not limited to, a hydroxyl group and an amino group. Examples of the fatty acid may include, but are not limited to, C8 to C20 fatty acids. In addition, examples of the C8 to C20 fatty acids may include, but are not limited to, lauric acid, capric acid, and stearic acid. In certain embodiments, the polyglycerol ester-based emulsifier may be formed by subjecting the first polyglycerol having the degree of polymerization ranging from 4 to 20 to an esterification reaction with the fatty acid (serving as the first acid).

Examples of the polyglycerol ester-based emulsifier may include, but are not limited to, polyglyceryl-10 laurate, polyglyceryl-4 laurate, polyglyceryl-4 caprate, and polyglyceryl-10 stearate. In certain embodiments, the polyglycerol ester-based emulsifier may be selected from the group consisting of the polyglyceryl-10 laurate having the hydrophilic-lipophilic balance value of 15.8, the polyglyceryl-4 laurate having the hydrophilic-lipophilic balance value of 12.2, and the polyglyceryl-4 caprate having the hydrophilic-lipophilic balance value of 16.4.

In certain embodiments, the polyglycerol ester-based coemulsifier may have a hydrophilic-lipophilic balance value ranging from 1 to 10. In certain embodiments, the polyglycerol ester-based coemulsifier may have a hydrophilic-lipophilic balance value greater than 1 and not greater than 8. In certain embodiments, the polyglycerol ester-based coemulsifier may have a hydrophilic-lipophilic balance value greater than 1 and smaller than 7. In certain embodiments, the polyglycerol ester-based coemulsifier may have a hydrophilic-lipophilic balance value greater than 1 and smaller than 6. In certain embodiments, the polyglycerol ester-based coemulsifier may have a hydrophilic-lipophilic balance value greater than 1 and smaller than 5. In certain embodiments, the polyglycerol ester-based coemulsifier may have a hydrophilic-lipophilic balance value greater than 1 and smaller than 4.

In certain embodiments, the second polyglycerol may have a degree of polymerization ranging from 2 to 8. In certain embodiments, the second polyglycerol may have a degree of polymerization ranging from 2 to 6. In certain embodiments, the second polyglycerol may have a degree of polymerization ranging from 2 to 4. In certain embodiments, the second acid may be a fatty acid or an aromatic acid. The fatty acid may or may not include a substituent. Examples of the substituent may include, but are not limited to, a hydroxyl group and an amino group. Examples of the fatty acid may include, but are not limited to, C14 to C20 fatty acids. An example of the C14 to C20 fatty acids may be, but is not limited to stearic acid. In certain embodiments, the polyglycerol ester-based coemulsifier may be formed by subjecting the polyol to an esterification reaction with the fatty acid (serving as a second acid) where the polyol is selected from the group consisting of the glycerol and the second polyglycerol having the degree of polymerization ranging from 2 to 10.

Examples of the polyglycerol ester-based coemulsifier may include, but are not limited to, polyglyceryl-2 isostearate, polyglyceryl-2 diisostearate, polyglyceryl-2 triisostearate, glyceryl stearate, and polyglyceryl-3 isostearate. In certain embodiments, the polyglycerol ester-based coemulsifier may be selected from the group consisting of the polyglyceryl-2 isostearate having the hydrophilic-lipophilic balance value of 6.5, the polyglyceryl-2 diisostearate having the hydrophilic-lipophilic balance value of 4.5, and the polyglyceryl-2 triisostearate having the hydrophilic-lipophilic balance value of 3.2.

<Oil-in-water sunscreen product>

Moreover, the present disclosure provides an oil-in-water sunscreen product including the aforementioned emulsion component. Examples of the oil-in-water sunscreen product may include, but are not limited to, a suncare product and a sunscreen cosmetic. Examples of the suncare product may include, but are not limited to, a sunscreen lotion, a sunscreen cream, a sunscreen spray, and a complete correction (CC) cream. An example of the sunscreen cosmetic may include, but is not limited to a blemish balm (BB) cream.

<Oil-in-water sunscreen lotion>

The present disclosure also provides an oil-in-water sunscreen lotion which includes the aforesaid emulsion component, a sunscreen agent, a fat, and water, and the emulsion component is present in an amount greater than 3 wt% and not greater than 30 wt% based on 100 wt% of the oil-in-water sunscreen lotion.

In certain embodiments, the emulsion component may be present in an amount greater than 3 wt% and not greater than 7 wt% based on 100 wt% of the oil-in-water sunscreen lotion.

The sunscreen agent may be selected from the group consisting of a chemical sunscreen agent, a physical sunscreen agent, and a combination thereof. Examples of the chemical sunscreen agent may include, but are not limited to, butyl methoxydibenzoylmethane and ethylhexyl methoxycinnamate. Examples of the physical sunscreen agent may include, but are not limited to, titanium dioxide and zinc oxide. In certain embodiments, the sunscreen agent may be present in an amount ranging from 0.1 wt% to 50 wt% based on 100 wt% of the oil-in-water sunscreen lotion. In certain embodiments, the sunscreen agent may be present in an amount ranging from 0.1 wt% to 30 wt% based on 100 wt% of the oil-in-water sunscreen lotion. In certain embodiments, the sunscreen agent may be present in an amount ranging from 0.1 wt% to 20 wt% based on 100 wt% of the oil-in-water sunscreen lotion.

Examples of the fat may include, but are not limited to, C12 to C15 alkyl benzoate, isononyl isononanoate, and caprylic/capric triglyceride. In certain embodiments, the fat may be present in an amount ranging from 0.1 wt% to 50 wt% based on 100 wt% of the oil-in-water sunscreen lotion. In certain embodiments, the fat may be present in an amount ranging from 0.1 wt% to 40 wt% based on 100 wt% of the oil-in-water sunscreen lotion. In certain embodiments, the fat may be present in an amount ranging from 0.1 wt% to 30 wt% based on 100 wt% of the oil-in-water sunscreen lotion. In certain embodiments, the fat may be present in an amount ranging from 0.1 wt% to 20 wt% based on 100 wt% of the oil-in-water sunscreen lotion.

In certain embodiments, the oil-in-water sunscreen lotion may further include a humectant. In certain embodiments, the humectant may be present in an amount ranging from 0.1 wt% to 30 wt% based on 100 wt% of the oil-in-water sunscreen lotion. In certain embodiments, the humectant may be present in an amount ranging from 0.1 wt% to 20 wt% based on 100 wt% of the oil-in-water sunscreen lotion. In certain embodiments, the humectant may be present in an amount ranging from 0.1 wt% to 10 wt% based on 100 wt% of the oil-in-water sunscreen lotion. Examples of the humectant may include, but are limited to, glycerol, propylene glycol, butylene glycol, pentylene glycol, hexylene glycol, caprylyl glycol, diglycerol, triglycerol, tetraglycerol, hexaglycerol, octaglycerol, and decaglycerol.

In certain embodiments, the oil-in-water sunscreen lotion may further include a thickening agent. Examples of the thickening agent may include, but are not limited to, xanthan gum, sodium alginate, a silicate, hydroxyethyl cellulose, carboxy methyl cellulose, and a carboxyvinyl polymer. The silicate may be magnesium aluminum silicate (Veegum®). In certain embodiments, the thickening agent may be present in an amount ranging from 0.01 wt% to 10 wt% based on 100 wt% of the oil-in-water sunscreen lotion. In certain embodiments, the thickening agent may be present in an amount ranging from 0.01 wt% to 5 wt% based on 100 wt% of the oil-in-water sunscreen lotion. In certain embodiments, the thickening agent may be present in an amount ranging from 0.01 wt% to 3 wt% based on 100 wt% of the oil-in-water sunscreen lotion. In certain embodiments, the thickening agent may be present in an amount ranging from 0.01 wt% to 2 wt% based on 100 wt% of the oil-in-water sunscreen lotion. In certain embodiments, the thickening agent may be present in an amount ranging from 0.01 wt% to 1 wt% based on 100 wt% of the oil-in-water sunscreen lotion.

In certain embodiments, the oil-in-water sunscreen lotion may further include a preservative. Examples of the preservative may include, but are not limited to, an organic acid, a salt, a polyol, a formaldehyde-releasing preservative, and a halide. The polyol may be ethylhexylglycerin. The halide may be chlorphenesin. In certain embodiments, the preservative may be present in an amount ranging from 0.001 wt% to 10 wt% based on 100 wt% of the oil-in-water sunscreen lotion. In certain embodiments, the preservative may be present in an amount ranging from 0.001 wt% to 5 wt% based on 100 wt% of the oil-in-water sunscreen lotion. In certain embodiments, the preservative may be present in an amount ranging from 0.001 wt% to 3 wt% based on 100 wt% of the oil-in-water sunscreen lotion. In certain embodiments, the preservative may be present in an amount ranging from 0.001 wt% to 2 wt% based on 100 wt% of the oil-in-water sunscreen lotion. In certain embodiments, the preservative may be present in an amount ranging from 0.001 wt% to 1 wt% based on 100 wt% of the oil-in-water sunscreen lotion.

The present disclosure will be further described by way of the following examples. However, it should be understood that the following examples are intended solely for the purpose of illustration and should not be construed as limiting the present disclosure in practice.

**EXAMPLES**

**Preparation of oil-in-water sunscreen lotion**

**Example 1**

2 wt% of butyl methoxydibenzoylmethane, 4 wt% of ethylhexyl methoxycinnamate (both serving as a chemical sunscreen agent), 6 wt% of titanium dioxide (serving as a physical sunscreen agent), 5 wt% of C12 to C15 alkyl benzoate (Patech Fine Chemicals Co., Ltd.; Model: Paester™ AB; INCI name: C12-C15 alkyl benzoate), 5 wt% of isononyl isononanoate, 5 wt% of caprylic/capric triglyceride (the three aforementioned ones serving as a fat), 4.2 wt% of polyglyceryl-10 laurate having a hydrophilic-lipophilic balance value of 15.8 (serving as a polyglycerol ester-based emulsifier), and 2.8 wt% of polyglyceryl-2 triisostearate having a hydrophilic-lipophilic balance value of 3.2 (serving as a polyglycerol ester-based coemulsifier) were mixed at 80°C, so as to form a first raw material composition. In addition, 62.2 wt% of water, 3 wt% of glycerol (serving as a humectant), 0.3 wt% of xanthan gum (serving as a thickening agent), and 0.5 wt% of a preservative (ACTIVON Co., Ltd.; Model: Activonol-PAFC) (containing phenoxyethanol, ethylhexylglycerin, and chlorphenesin) were mixed at 80°C, so as to form a second raw material composition. After that, the first raw material composition was slowly added into the second raw material composition, followed by thoroughly stirring to form a mixture well mixed. Subsequently, the mixture was subjected to a homogenization treatment utilizing a homogenizer at a speed of 5000 rpm for 5 minutes, and was then cooled down to 40°C with gentle stirring, thereby obtaining an oil-in-water sunscreen lotion.

**Example 2 and Comparative Examples 1 to 4**

The procedures for preparing the oil-in-water sunscreen lotion in each of Example 2 and a sunscreen in each of Comparative Examples 1 to 4 were similar to those of Example1, except that the amount of each content therein was varied as shown in Table 1 and 2 below.

Table 1

| | | Content (wt%) | | Example | |
|---|---|---|---|---|---|
| | | | | 1 | 2 |
| First raw material composition | Chemical sunscreen agent | Butyl methoxydibenzoylmethane | | 2 | 2 |
| | | Ethylhexyl methoxycinnamate | | 4 | 4 |
| | Physical sunscreen agent | Titanium dioxide | | 6 | 6 |
| | Fat | C12 to C15 alkyl benzoate | | 5 | 5 |
| | | Isononyl isononanoate | | 5 | 5 |
| | | Caprylic/capric triglyceride | | 5 | 5 |
| | Emulsion component | Polyglycerol ester-based emulsifier | Polyglyceryl-10 laurate having a HLB value of 15.8 | 4.2 | 3.5 |
| | | Polyglycerol ester-based coemulsifier | Polyglyceryl-2 triisostearate having a HLB value of 3.2 | 2.8 | 3.5 |
| | | Total amount | | 7 | 7 |
| Second raw material composition | | Water | | 62.2 | 62.2 |
| | Humectant | Glycerol | | 3 | 3 |
| | Thickening agent | Xanthan gum | | 0.3 | 0.3 |
| | Preservative | Activonol-PAFC | | 0.5 | 0.5 |

Table 2

| Content (wt%) | | | Comparative Example | | | |
|---|---|---|---|---|---|---|
| | | | 1 | 2 | 3 | 4 |
| First raw material composition | Chemical sunscreen agent | Butyl methoxydibenzoylmethane | 2 | 2 | 2 | 2 |
| | | Ethylhexyl methoxycinnamate | 4 | 4 | 4 | 4 |
| | Physical sunscreen agent | Titanium dioxide | 6 | 6 | 6 | 6 |
| | Fat | C12 to C15 alkyl benzoate | 5 | 5 | 5 | 5 |
| | | Isononyl isononanoate | 5 | 5 | 5 | 5 |
| | | Caprylic/capric triglyceride | 5 | 5 | 5 | 5 |
| | Emulsion component | Polyglycerol ester-based emulsifier | Polyglyceryl-10 laurate having a HLB value of 15.8 | 7 | 1.8 | 0 | 6.3 |
| | | Polyglycerol ester-based coemulsifier | Polyglyceryl-2 triisostearate having a HLB value of 3.2 | 0 | 1.2 | 7 | 0.7 |
| | | Total amount | | 7 | 3 | 7 | 7 |
| Second raw material composition | Water | | 62.2 | 62.2 | 62.2 | 62.2 |
| | Humectant | Glycerol | 3 | 3 | 3 | 3 |
| | Thickening agent | Xanthan gum | 0.3 | 0.3 | 0.3 | 0.3 |
| | Preservative | Activonol-PAFC | 0.5 | 0.5 | 0.5 | 0.5 |

## Property Evaluation

### A. Determination of hydrophilic-lipophilic balance (HLB) value:

The HLB value of each of polyglycerol ester-based emulsifier was calculated by using the following Equation (I):

$$A = (B/C) \times 20 \qquad (I)$$

where A= HLB value of polyglycerol ester-based emulsifier
B= Amount of first polyglycerol having a degree of polymerization ranging from 4 to 20
C= Total amount of first polyglycerol having a degree of polymerization ranging from 4 to 20 and fatty acid (serving as a first acid)

The HLB value of each of polyglycerol ester-based coemulsifier was calculated by using the following Equation (II):

$$D = (E/F) \times 20 \qquad (II)$$

where D= HLB value of polyglycerol ester-based coemulsifier
E= Amount of glycerol or second polyglycerol having a degree of polymerization ranging from 2 to 10 (serving as a polyol)
F= Total amount of glycerol/second polyglycerol having a degree of polymerization ranging from 2 to 10 and fatty acid (serving as a second acid)

**B. Analysis of emulsification:**

Each of the oil-in-water sunscreen lotions of Examples 1 to 2 and Comparative Examples 1 to 4 was observed with naked eyes to see whether or not stratification or unevenness in the form of an oil slick would occur therein. If no stratification and no oil slick was observed, then the emulsification was complete (denoted by "O" in Table 3). If stratification or oil slick was observed, then the emulsification was incomplete or no emulsification occurred (denoted by "X" in Table 3). The results are shown in Table 3 below.

**C. Analysis of emulsification type:**

An appropriate amount of each of the oil-in-water sunscreen lotion of Examples 1 and 2 and Comparative Examples 1 to 4 was dropped into water and then was observed with naked eyes to see whether or not the oil-in-water sunscreen lotion may be uniformly dispersed in the water. If the oil-in-water sunscreen lotion could be evenly dispersed in the water, the emulsification type was in an oil-in-water form (O/W form). On the other hand, if an agglomeration, which may float on the surface of the water or sink to the bottom of the water, was detected, that is to say, the oil-in-water sunscreen lotion could not be dispersed in the water, the emulsification type was in a water-in-oil form (W/O form). The results are shown in Table 3 below.

**D. Evaluation of sun protection factor (SPF) and water resistance retention (WRR) percentage:**

39 mg to 40 mg of each of the oil-in-water sunscreen lotion of Examples 1 and 2 and the sunscreen of Comparative Examples 1 and 4 was respectively coated onto a poly(methyl methacrylate) plate (Schönberg), so as to form a coating having 0.7 mg to 1.3 mg of the oil-in-water sunscreen lotion/the sunscreen per unit area ($cm^2$) thereon, thereby obtaining 6 test samples. Subsequently, each of the test samples was left standing in the dark for 15 minutes, and then subjected to a measurement with reference to the standard protocol of ISO 24443 (2012), Cosmetics-Determination of sunscreen UVA photoprotection in vitro, utilizing an ultraviolet spectrometer (Shimadzu; Model: UV-2600i), thereby obtaining a first SPF. After that, each of the test samples was immersed in 3 liters of water, followed by stirring the water utilizing a stirrer at a speed of 5000 rpm for 15 minutes, then was taken out from the water and subsequently a residual water remaining thereon was removed. Next, each of the test samples was subjected to a measurement with reference to the aforesaid standard protocol of ISO 24443 (2012) utilizing the ultraviolet spectrometer so as to obtain a second SPF. The WRR percentage of each of the test samples was calculated using the following Equation (III):

$$G=[(H-1)/(I-1)]\times100\% \qquad (III)$$

where G= WRR percentage

H= Second SPF

I= First SPF

The result are shown in Table 3 below.

Table 3

| | Example | | Comparative Example | | | |
|---|---|---|---|---|---|---|
| | 1 | 2 | 1 | 2 | 3 | 4 |
| Emulsification | O | O | O | X | O | O |
| Emulsification type | O/W form | O/W form | O/W form | * | W/O form | O/W form |
| First SPF | 59.5 | 43.29 | 32.12 | - | - | 39.88 |
| Second SPF | 45.6 | 28.24 | 19.6 | - | - | 8.8 |
| WRR (%) | 75.8 | 64.4 | 59.8 | - | - | 20.1 |
| *: cannot be emulsified | | | | | | |

As shown in Table 3, by using the polyglycerol ester-based emulsifier and the polyglycerol ester-based coemulsifier in the emulsion component, the oil-in-water sunscreen lotion prepared from the emulsion component may obtain a good sun protection factor and exhibit good water resistance effect. Moreover, polyglycerin-10 (serving as the first polyglycerol) and lauric acid (serving as the first acid) in the polyglyceryl-10 laurate having a HLB value of 15.8 (i.e., the polyglycerol ester-based emulsifier), and diglycerin (serving as the second polyglycerol) and isostearic acid (serving as the second acid) in the polyglyceryl-2 triisostearate having a hydrophilic-lipophilic balance value of 3.2 (i.e., the polyglycerol ester-based coemulsifier) are all natural ingredients, so the emulsion component may not only meet the current market demand for natural ingredients, but is also beneficial to the sustainable development of the cosmetic market.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. An emulsion component, **characterized by**:

    a polyglycerol ester-based emulsifier having a hydrophilic-lipophilic balance value greater than 10, the polyglycerol ester-based emulsifier being formed by reacting a first polyglycerol having a degree of polymerization ranging from 4 to 20 with a first acid, and
    a polyglycerol ester-based coemulsifier having a hydrophilic-lipophilic balance value not greater than 10, the polyglycerol ester-based coemulsifier being formed by reacting a polyol with a second acid, the polyol being selected from the group consisting of a glycerol and a second polyglycerol having a degree of polymerization ranging from 2 to 10;
    wherein a weight ratio of the polyglycerol ester-based emulsifier and the polyglycerol ester-based coemulsifier ranges from 0.75 to 8.

2. The emulsion component as claimed in claim 1, wherein the polyglycerol ester-based emulsifier has a hydrophilic-lipophilic balance value greater than 10 and not greater than 20.

3. The emulsion component as claimed in any one of claims 1 and 2, wherein the polyglycerol ester-based coemulsifier has a hydrophilic-lipophilic balance value ranging from 1 to 10.

4. The emulsion component as claimed in any one of claims 1 to 3, wherein the weight ratio of the polyglycerol ester-based emulsifier and the polyglycerol ester-based coemulsifier ranges from 0.75 to 7.

5. The emulsion component as claimed in any one of claims 1 to 4, wherein the weight ratio of the polyglycerol ester-based emulsifier and the polyglycerol ester-based coemulsifier ranges from 0.75 to 6.

6. An oil-in-water sunscreen product, **characterized by** an emulsion component as claimed in any one of claims 1 to 5.

7. The oil-in-water sunscreen product as claimed in claim 6, which is selected from the group consisting of a suncare product and a sunscreen cosmetic.

8. An oil-in-water sunscreen lotion, **characterized by** an emulsion component as claimed in any one of claims 1 to 5, a sunscreen agent, a fat, and water, wherein the emulsion component is present in an amount greater than 3 wt% and not greater than 30 wt% based on 100 wt% of the oil-in-water sunscreen lotion.

Europäisches
Patentamt

European
Patent Office

Office européen
des brevets

# EUROPEAN SEARCH REPORT

**Application Number**

EP 23 19 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 102 311 828 B1 (CHOICE INT TRADING CORPORATION [KR]) 12 October 2021 (2021-10-12) * the whole document * * examples 1-3 * | 1-8 | INV. A61K8/37 A61Q17/04 |
| X | WO 2012/167901 A2 (BEIERSDORF AG [DE]; VON THADEN STEFANIE [DE]; KOEHLER MANUELA [DE]) 13 December 2012 (2012-12-13) * the whole document * * claims 1,3,6,7 * | 1-8 | |
| X | WO 2021/140913 A1 (SHISEIDO CO LTD [JP]) 15 July 2021 (2021-07-15) * Examples * | 1 | |
| T | DAMIAN DIONA L.: "Photoprotective effects of nicotinamide", PHOTOCHEMICAL & PHOTOBIOLOGICAL SCIENCES, vol. 9, no. 4, 1 April 2010 (2010-04-01), pages 578-585, XP093006365, GB ISSN: 1474-905X, DOI: 10.1039/b9pp00146h Retrieved from the Internet: URL:https://link.springer.com/content/pdf/10.1039/b9pp00146h.pdf?pdf=button> | | |
| T | Anonymous: "NIKKOL Nikkoguard DL(Glyceryl Caprate, Polyglyceryl-2 Laurate, Polyglyceryl-10 Laurate) ¦ Chemical-Navi Product Search <chemical-navi.com>", , 31 December 2021 (2021-12-31), XP93109634, Retrieved from the Internet: URL:https://www.chemical-navi.com/en/product-search/detail270.html [retrieved on 2023-12-06] | | |

**TECHNICAL FIELDS SEARCHED (IPC)**

A61Q
A61K

-/--

The present search report has been drawn up for all claims

| Place of search | Date of completion of the search | Examiner |
|---|---|---|
| Munich | 14 January 2024 | Skulj, Primoz |

EPO FORM 1503 03.82 (P04C01)

page 1 of 2

Europäisches
Patentamt

European
Patent Office

Office européen
des brevets

# EUROPEAN SEARCH REPORT

**Application Number**

EP 23 19 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | CN 114 948 766 A (SHANGHAI ZHENCHEN COSMETICS CO LTD) 30 August 2022 (2022-08-30) * the whole document * * see also WPI abstract * | 1 | |

TECHNICAL FIELDS
SEARCHED      (IPC)

The present search report has been drawn up for all claims

| Place of search | Date of completion of the search | Examiner |
|---|---|---|
| Munich | 14 January 2024 | Skulj, Primoz |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 19 2091

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2024

| Patent document cited in search report | | Publication date | Patent family member(s) | | Publication date |
|---|---|---|---|---|---|
| KR 102311828 | B1 | 12-10-2021 | NONE | | |
| WO 2012167901 | A2 | 13-12-2012 | BR 112013031085 | A2 | 06-09-2016 |
| | | | CN 104053427 | A | 17-09-2014 |
| | | | CN 108635244 | A | 12-10-2018 |
| | | | DE 102011077037 | A1 | 13-12-2012 |
| | | | EP 2775997 | A2 | 17-09-2014 |
| | | | ES 2753583 | T3 | 13-04-2020 |
| | | | US 2014140940 | A1 | 22-05-2014 |
| | | | WO 2012167901 | A2 | 13-12-2012 |
| WO 2021140913 | A1 | 15-07-2021 | CN 114929186 | A | 19-08-2022 |
| | | | JP 2021109830 | A | 02-08-2021 |
| | | | US 2023048913 | A1 | 16-02-2023 |
| | | | WO 2021140913 | A1 | 15-07-2021 |
| CN 114948766 | A | 30-08-2022 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103585040 B